(19) Europäisches Patentamt
European Patent Office
Office européen des brevets

(11) Publication number: **0 255 384 B1**

# (12) EUROPEAN PATENT SPECIFICATION

(45) Date of publication of patent specification: **16.10.91**

(51) Int. Cl.5: **C07C 317/14**, C08G 63/00, C08G 69/00, C08G 73/10

(21) Application number: **87306755.7**

(22) Date of filing: **30.07.87**

## (54) 4,4-bis-(carboxyphenylsulfonyl)diphenyl ethers, process for their preparation and their use.

(30) Priority: **30.07.86 JP 179606/86**
**02.09.86 JP 206600/86**

(43) Date of publication of application:
**03.02.88 Bulletin 88/05**

(45) Publication of the grant of the patent:
**16.10.91 Bulletin 91/42**

(84) Designated Contracting States:
**DE FR GB**

(56) References cited:
**US-A- 4 102 905**

**CHEMICAL ABSTRACTS, vol. 74, no. 22, 31st May 1971, page39, abstract no. 113020c, Columbus, Ohio, US; S.S. GITIS et al.: "p,p'-Bis(p-carboxyphenylsulfonyl)diphenyl oxide and some of its derivatives as monomers for the preparation of heat-resistant fibers", & KHIM. VOLOKNA 1971, (1), 45-7**

(73) Proprietor: **Tonen Corporation**
**1-1 Hitotsubashi, 1-Chome Chiyoda-Ku**
**Tokyo 100(JP)**

(72) Inventor: **Imai, yoichi**
**4-6 Nishitsurugaoka 1-chome Ooi-machi**
**Iruma-gun Saitama(JP)**
Inventor: **Niwa, Tadashi**
**4-4-421 Nishitsurugaoka 1-chome, Ooi-Machi**
**Iruma-gun Saitama(JP)**
Inventor: **Kato, Masayuki**
**4-4-443 Nishitsurugaoka 1-chome, Ooi-Machi**
**Iruma-gun Saitama(JP)**
Inventor: **Taguchi, Yoshio**
**4-4-424 Nishitsurugaoka 1-chome, Ooi-Machi**
**Iruma-gun Saitama(JP)**
Inventor: **Imai, Chihiro**
**49-5 Utsukushigaoka 4-chome Midori-ku**
**Yokohama-shi Kanagawa-ken(JP)**

(74) Representative: **Baverstock, Michael George Douglas et al**
**BOULT, WADE & TENNANT 27 Furnival Street**
**London, EC4A 1PQ(GB)**

**EP 0 255 384 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for producing aromatic carboxylic acids having sulfonyl groups, and, more particularly, to aromatic carboxylic acids having sulfonyl groups which are used in producing useful polymers such as polyesters, polyamides and polyimides.

Many aromatic compounds having functional groups such as carboxyl and imide groups have been known as raw materials for heat-resistant polymers. For example, pyromellitic acid (anhydride) from which polyimides represented by Kapton (Registered Trade Mark) are produced, and aromatic bis (ether acid anhydrides) from which polyether imides are produced (Japanese Patent Laid-Open Publication No. 24242/1975) have been known in the art.

While polyimides obtained by using pyromellitic acid (anhydride) as the raw material have excellent heat resistance, their moldability is inferior. The polyetherimides obtained by using the aromatic bis (ether acid anhydride) as the raw material are inferior in heat resistance.

Thus, the development of aromatic carboxylic acids from which polyimides having an improved balance between heat resistance and moldability is desirable.

On the other hand, polyesters obtained by the polycondensation of a dibasic acid with a divalent alcohol wherein the main chain is attached by ester linkage have been widely used as polymeric materials for synthetic fibers or as polymeric materials for films. Further it is well known that polyamides useful as polymeric materials for synthetic fibers can be produced from dibasic acids and diamines.

Thus, dibasic acids are compounds which are extremely useful as starting materials in producing polyesters or polyamides. Terephthalic acid obtained by the oxidation reaction of para-xylene and isophthalic acid obtained by the oxidation reaction of meta-xylene represented by the reaction schemes as described hereinafter are widely known as dibasic acids from which polymers as described above are produced.

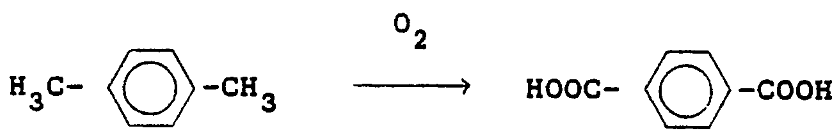


Terephthalic acid

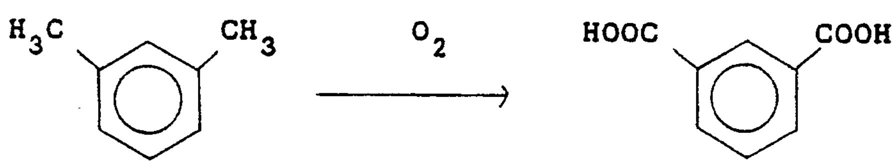


Isophthalic acid

Techniques for producing polymers such as polyesters and polyamides using terephthalic acid or isophthalic acid as the starting material have been thoroughly studied. It is known that one polymer obtained is inferior in heat resistance and that the other polymer obtained is inferior in processability.

US-A-4,102,905 discloses that 4,4'-bis (3,4-dicarboxyphenyl sulfonyl) diphenyl ether is useful as a curing agent for epoxy resins and as an intermediate for polyamides and polyesters. However, according to the U.S. patent the diphenyl ether cannot be obtained on an industrial scale.

Accordingly, the development of aromatic carboxylic acids from which polyesters, polyamides or polyimides can be produced is desirable.

In view of the foregoing, the Applicants have carried out extensive studies to produce polyesters and polyamides by the provision of aromatic carboxylic acids. It has now been found that aromatic carboxylic acids containing sulfonyl groups may be prepared by oxidizing bis[4-(4-methylphenylsulfonyl)-phenyl]ethers obtained from paratoluenesulfonyl chlorides and diphenyl ethers, and related compounds thereof. The

present invention is based on this discovery.

An object of the present invention is to provide a new process for producing aromatic carboxylic acids containing sulfonyl groups which are useful in producing polymers such as polyesters, polyamides and polyimides.

According to the present invention there is provided a process for producing an aromatic carboxylic acid represented by the following formula [V]:

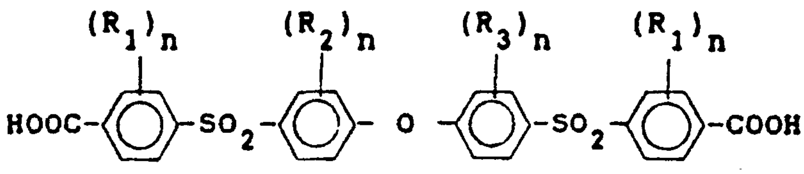

[V]

wherein $R_1$, $R_2$ and $R_3$, which may be the same or different, are hydrogen, a lower alkyl group containing from 1 to 6 carbon atoms or a carboxyl group; and n is the number of substituents other than hydrogen and is an integer of from 0 to 4, which process comprises the step of oxidizing a compound represented by the following general formula [IV]:

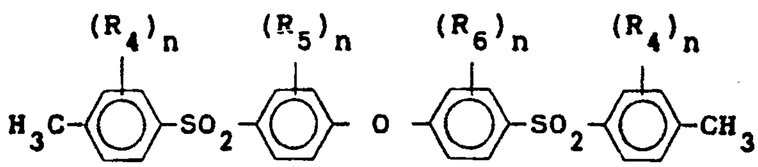

[IV]

wherein n is as defined above; $R_4$ is hydrogen or a methyl group; and $R_5$ and $R_6$, which may be the same or different, are hydrogen, or a lower alkyl containing from 1 to 6 carbon atoms, wherein the step of oxidizing is performed with molecular oxygen in a liquid phase in an aliphatic carboxylic acid solvent in the presence of a cobalt-maganese-bromine catalyst.

A process for producing the novel aromatic carboxylic acids containing sulfonyl groups according to the present invention as represented by the following formula [V] comprises oxidizing a compound represented by the following formula [IV] which may be obtained by reacting paratoluenesulfonyl chlorides represented by the following formula [II] with diphenyl ethers represented by the following general formula [III]:

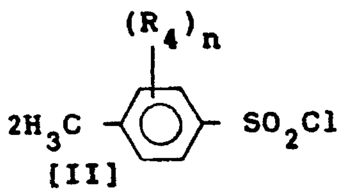

[II]

+

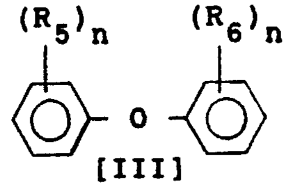

[III]

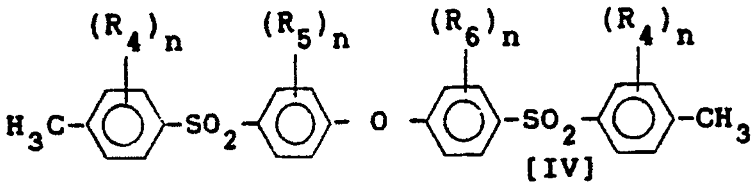

[IV]

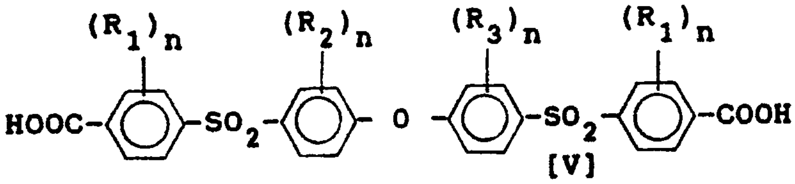

[V]

wherein $R_1$ is hydrogen, a lower alkyl group containing from 1 to 6 carbon atoms or a carboxyl group; $R_2$ and $R_3$ are the same as above: $R_4$ is hydrogen or a methyl group: $R_5$ and $R_6$ are hydrogen, a lower alkyl containing from 1 to 6 carbon atoms; and n is the number of substituents other than hydrogen and is an integer of from 0 to 4. Preferably $R_1$ is hydrogen.

In producing the aromatic carboxylic acid represented by the formula [V] described above by oxidizing the compound represented by the formula [iv] described above, the compound represented by the formula [iv] is oxidized with molecular oxygen in a liquid phase in an aliphatic carboxylic acid solvent in the presence of a cobalt-manganese-bromine catalyst.

According to the present invention, the aromatic carboxylic acids containing sulfonyl groups from which polyesters and polyamides may be produced can be obtained on an industrial scale without using any expensive oxidizing reagent.

In the drawings, Fig. 1 is a DTA analysis chart of 4,4'-bis(4-carboxyphenylsulfonyl)diphenyl ether prepared according to the present invention and

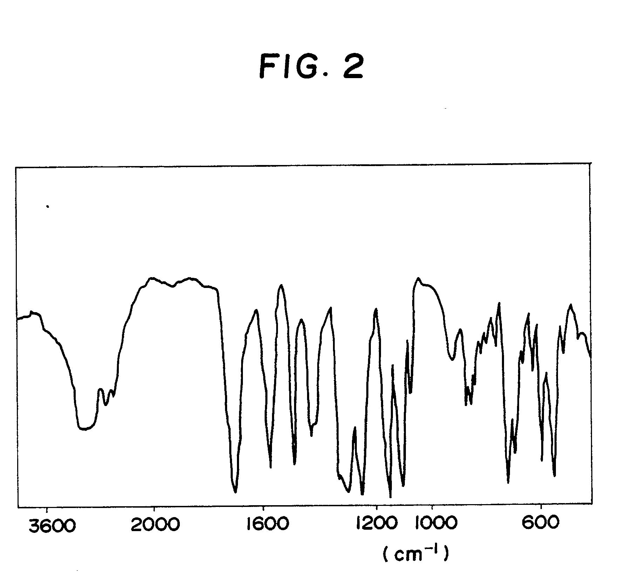

Fig. 2 is the infrared analysis chart of this compound.

A process for producing aromatic carboxylic acid containing sulfonyl groups according to the present invention will now be fully described.

The preferred aromatic carboxylic acids containing sulfonyl groups according to the present invention are represented by the following general formula [I]:

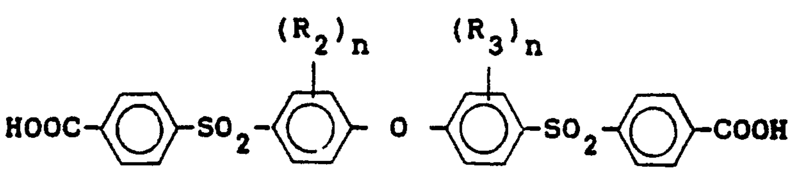

[I]

wherein $R_2$ and $R_3$, which may be the same or different, are hydrogen, a lower alkyl group containing from 1 to 6 carbon atoms or a carboxyl group; and n is the number of substituents other than hydrogen and is an integer of from 0 to 4.

Of such aromatic carboxylic acids, a compound wherein $R_1$, $R_2$ and $R_3$ are all hydrogen is particularly preferred. When $R_1$, $R_2$ and $R_3$ are all hydrogen, this aromatic carboxylic acid is 4,4'-bis(4-carboxyphenylsulfonyl)diphenyl ether.

The aromatic carboxylic acids as described above can be used to produce polymers useful as synthetic fibers or the like, for example, by the polycondensation with ethylene glycol.

The structure of the aromatic carboxylic acids represented by the general formula [I] described above is determined by IR, elementary analysis, melting point or the like.

A process for producing the aromatic carboxylic acids represented by the general formula [V] will now be described.

First, a para-toluenesulfonyl chloride represented by the following general formula [III] is reacted with a diphenyl ether represented by the following general formula [III], for example, in the presence of a Lewis acid such as aluminium trichloride, to produce a bis[4-(4-methylphenylsulfonyl)phenyl] ether represented by the following general formula [IV].

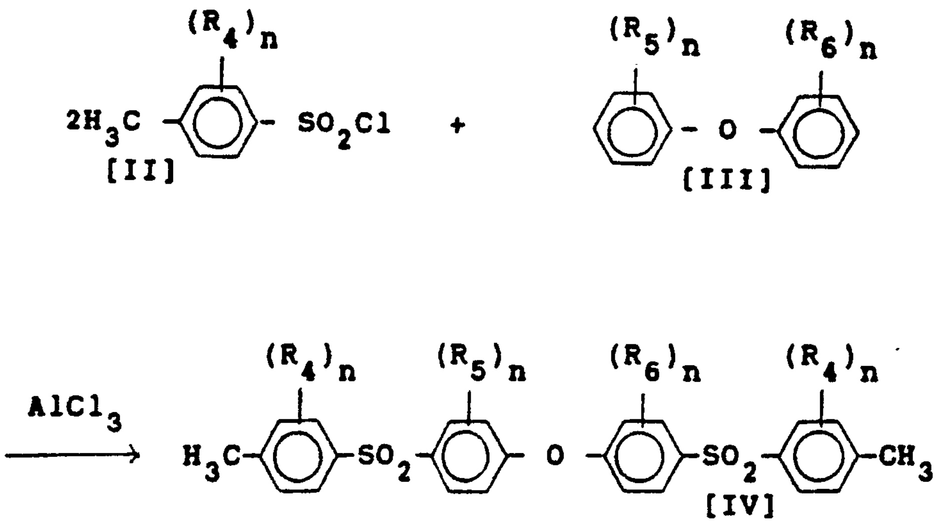


[II] [III] [IV]

The reaction of para-toluenesulfonyl chloride represented by the general formula [II] with a diphenyl ether represented by the general formula [III] is carried out by contacting both compounds with stirring for from 0.5 to 50 hours at a temperature of from -70 °C to +250 °C in the presence of a catalyst selected from Lewis acids such as aluminum trichloride, boron trifluoride and ferric chloride; proton acids such as sulfuric acid, methanesulfonic acid and trifluoromethanesulfonic acid; and metal powders such as iron powder and aluminum powder. The paratoluenesulfonyl chloride is used in an amount of at least two moles, preferably from 2 to 100 moles per mole of the diphenyl ether. It is preferable that the amount of the catalyst used is from 0.01 to 20 moles per mole of the diphenyl ether.

The term "diphenyl ether", as used herein, refers to diphenyl ether and derivatives thereof. In addition, the term "para-toluenesulfonyl chloride", as used herein, refers to para-toluenesulfonyl chloride and derivatives thereof.

While the reaction described above can also be carried out without using any solvent, it can additionally be carried out in a solvent selected from halogenated hydrocarbons such as methyl chloride, methylene chloride, chloroform, carbon tetrachloride, dichloroethane, trichloroethane, ethylene dichloride, ethylene trichloride, bromoform, chlorobenzene, and dichlorobenzene; nitro compounds such as nitromethane, nitroethane, and nitrobenzene; and aliphatic or alicyclic hydrocarbons such as carbon disulfide, hexane, heptane and cyclohexane.

After the completion of the reaction, the aromatic compounds represented by the general formula [IV] can be synthesized by adding water to the reaction system to decompose the catalyst, and distilling off excess paratoluenesulfonyl chlorides by steam distillation or the like. The purity of the compounds can be increased by recrystallization with an alcoholic aqueous solution or the like.

Optionally, the aromatic compounds represented by the general formula [IV] described above can also be produced by the reaction of dichlorosulfonyldiphenyl ethers with xylenes.

This reaction is represented by, for example, the following scheme:

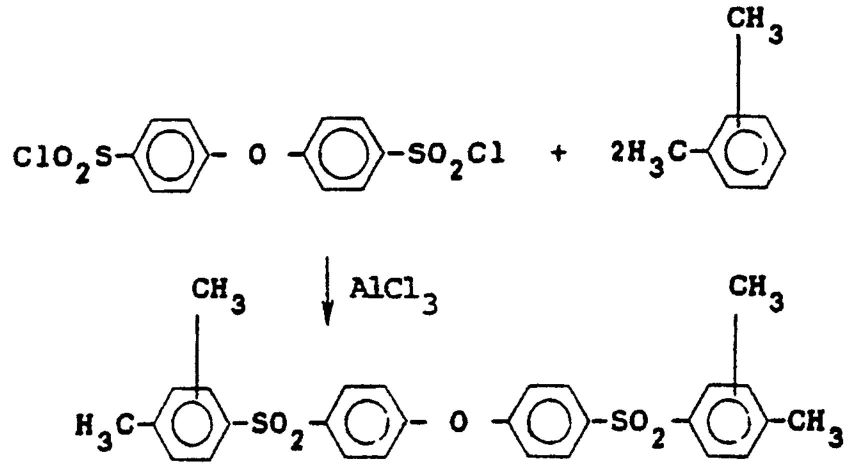


[IV]

Aromatic carboxylic acids, represented by the general formula [V] are then obtained by oxidizing both terminal methyl groups of the thus-obtained bis[4-(4-methylphenylsulfonyl) phenyl]ethers represented by the general formula [IV]:

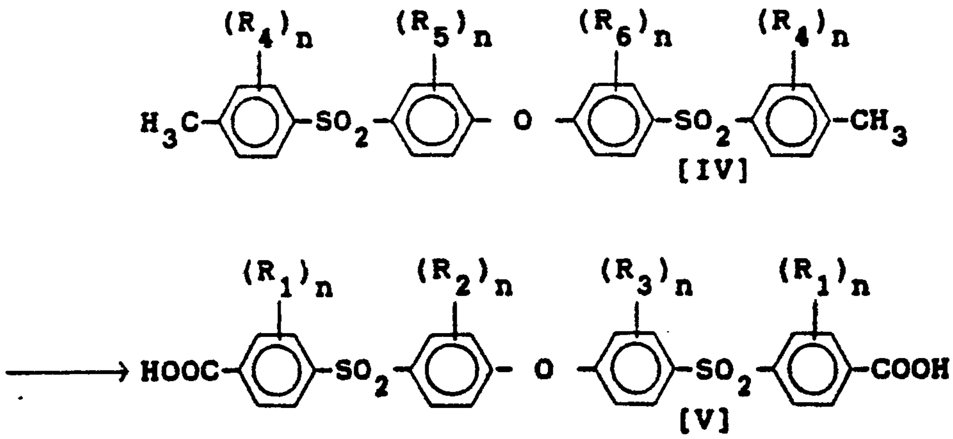

[IV]

[V]

The aromatic compound represented by the general formula [IV] is oxidized with molecular oxygen in a liquid phase in an aliphatic carboxylic acid solvent in the presence of a cobalt-manganese-bromine catalyst to obtain aromatic carboxylic acids represented by the general formula [V] described above.

Examples of the aliphatic carboxylic acids for use as the reaction solvent include acetic acid, propionic acid, butyric acid and valeric acid. Of the above, acetic acid is particularly preferred. Such aliphatic carboxylic acids may contain a small amount of water. Optionally, organic solvents such as aldehydes, ketones and alcohols can also be incorporated in the aliphatic carboxylic acids.

It is preferable that such solvents are used in an amount of from 1 to 10 parts by weight based on 1 part by weight of the aromatic compounds which are reactants.

Cobalt-manganese-bromine catalysts are used as the catalysts for the above reaction. Cobalt and manganese are usually used as metal salts soluble in the reaction solvent, including inorganic salts, acetates and naphthenates. Examples of salts of cobalt and manganese for use herein include cobalt acetate, cobalt bromide, cobalt naphthenate, manganese acetate, manganese bromide and manganese naphthenate. Also, bromine may be added to the reaction system as inorganic bromine compounds such as hydrogen bromide, sodium bromide and potassium bromide or as organic bromine compounds such as tetrabromoethane, or in the form of cobalt bromide or manganese bromide.

It is preferable that the cobalt salt is used in an amount of from 0.01% to 0.5% by weight (on a metal basis) based on the weight of the solvent, and that the manganese salt is used in an amount of from 0.005% to 0.2% by weight (on a metal basis) based on the weight of the solvent. Further, it is preferable that the bromine compound is used in an amount of from 0.1% to 0.5% by weight based on the weight of

the solvent.

In the oxidation reaction, molecular oxygen may be used as the oxygen source. Pure oxygen or air may be used to provide molecular oxygen.

It is desirable that the reaction temperature is from 50° to 250° C, preferably from 80° C to 210° C. The reaction can be carried out either under atmospheric pressure or under elevated pressure such as up to about 30 atm.

The aromatic carboxylic acids which are the reaction products can be obtained, for example, by cooling the reaction mixture, adding water to the reaction mixture, dissolving the deposited solid in an alkaline aqueous solution and then acidifying this solution to deposit a solid.

According to the present invention, there are effectively obtained aromatic carboxylic acids containing sulfonyl groups from which polymers such as polyesters, polyamides and polyimides may be produced.

While the present invention is illustrated by the following Examples, it is not limited thereto.

EXAMPLE 1

A 2-liter 4-necked flask was charged with 75.5 grams of diphenyl ether, 118.4 grams of aluminum trichloride and 400 milliliters of nitromethane, and the reaction mixture was stirred while cooling to 0° C in an ice bath. To this were gradually added 253.6 grams of solid para-toluenesulfonic acid chloride under nitrogen. After the total amount was added, the reaction was continued for 20 hours while cooling the reaction mixture in an ice bath. Thereafter, water was added to the reaction mixture to stop the reaction. Chloroform was added to the resulting reaction mixture to extract and activated charcoal was added to the organic layer and filtration was carried out. Nitromethane and chloroform were removed from the filtrate to recover 150 grams of solid.

This recovered solid was dissolved in an ethanol/water mixture (the weight ratio of ethanol:water was 6:4) to recrystallize it therefrom to obtain light cream-coloured crystals.

The melting point of these crystals was from 167° C to 168° C.

The results of IR analysis of the crystals are shown hereinafter.

3025~3075 $cm^{-1}$; CH stretching of aromatics;

2910~2970 $cm^{-1}$; CH stretching of aliphatics;

1485 and 1580$cm^{-1}$; benzene rings; 1160 and 1320 $cm^{-1}$; -$SO_2$-; and 1240 $cm^{-1}$; -O-.

Further, the results of $^1$H-NMR analysis of the crystals are shown hereinafter.

$\delta$ = 2.40 singlet (6H)

$\delta$ = 6.70-8.20 multiplet (16H)

Furthermore, the results of elementary analysis of the crystals are shown hereinafter.

| | C | H | S |
|---|---|---|---|
| Found : | 65.4% | 4.8% | 13.3% |
| Calcd.: | 65.25% | 4.63% | 13.40% |

From these results, the crystalline solid obtained in the reaction described above was confirmed to be 4,4'-bis(4-methylphenylsulfonyl) diphenyl ether represented by the following formula:

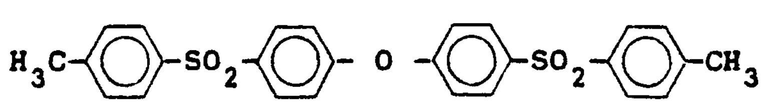

The oxidation of 4,4'-bis(4-methylphenylsulfonyl)diphenyl ether obtained as described above was then carried out as follows (not in accord with the process of the invention):

A 500 ml 4-necked flask equipped with a stirrer and condenser was charged with 4 grams of 4,4'-bis(4-methylphenylsulfonyl)diphenyl ether, and it was dissolved in 130 milliliters of pyridine. To this were added KOH (0.5 gram), water (33 milliliters) and 10 grams of $KMnO_4$, and the reaction was carried out while maintaining the temperature at a reflux temperature with stirring/ $KMnO_4$ (15 grams) was added in a divided manner (3 times), and the reflux was continued until the purple colour of $KMnO_4$ disappeared.

The resulting reaction mixture was cooled, and the solid matter was removed by filtration. After the solvent was removed from the filtrate, 100 milliliters of a 10% NaOH aqueous solution were added thereto to obtain an alkaline aqueous solution of the product. This solution was acidified with 10% HCl to deposit a white solid (4.1 grams). This solid (3 grams) was washed with hot acetic acid and a white crystalline solid (1.3 gram) was obtained.

The melting point of the resulting crystals was measured by DTA, an a DTA curve is shown in Fig. 1. As can be seen from the drawing, the melting point Tm of the crystals is 406 °C.

An IR analysis chart of the crystals is shown in Fig. 2.

As can be seen from this IR chart, absorption was observed at 3600-2400 $cm^{-1}$ and 1700 $cm^{-1}$ (-COOH), 1580 and 1490 $cm^{-1}$ (benzene rings), 1250 $cm^{-1}$ (-O-), and 1325 $cm^{-1}$ ($-SO_2-$).

Further, the results of elementary analysis of the crystals are shown below:-

| | C | H | S |
|---|---|---|---|
| Found : | 58.2% | 3.2% | 11.8% |
| Calcd.: | 57.99% | 3.37% | 11.91% |

From these results, the crystals obtained in the reaction described above was confirmed to be 4,4 bis-(4-carboxyphenylsulfonyl)diphenyl ether represented by the following formula:

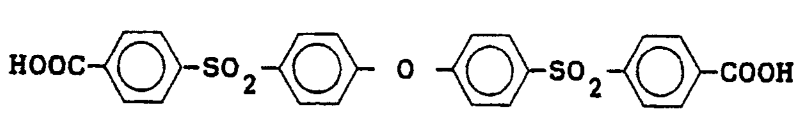

EXAMPLE 2

Five grams of p,p'-dichlorosulfonyldiphenyl ether were dissolved in 30 milliliters of ortho-xylene, and the solution was cooled in an ice bath. To this were added 6.38 grams of anhydrous aluminum chloride solid and the reaction mixture was stirred for 12 hours at room temperature. Thereafter, 100 milliliters of water were added to stop the reaction. Excess ortho-xylene was then removed by steam distillation to obtain 6.7 grams of a brown solid. This solid was recrystallized from an ethanol/water mixture (6:4) to obtain light brown crystals. The melting point of these crystals was 89 °C.

The results of IR analysis of the crystals were as follows:
1240 $cm^{-1}$; -O-; 1315 and 1150 $cm^{-1}$; $-SO_2-$;
2910-2970 $cm^{-1}$; CH stretching of aliphatics;
3025-3075 $cm^{-1}$; CH stretching of aromatics; and
1475 and 1570 $cm^{-1}$: benzene rings.

Further, the results of $^1H$-NMR analysis were as follows:
δvalue (ppm) 2.22 singlet (6H)
(TMS basis) 2.36 singlet (6H)
7.26-8.30 multiplet (14H)

Furthermore, the results of elementary analysis were as follows:
Anal. calcd. for $C_{28}H_{26}O_3S_2$: C, 66.38%; H, 5.17%.
Found: C, 66.78%; H, 5.30%.

From the results described above, the material obtained in this example was confirmed to be 4,4-bis-(3,4-dimethylphenylsulfonyl)diphenyl ether (hereinafter referred to as DXDE) having the following structural formula:

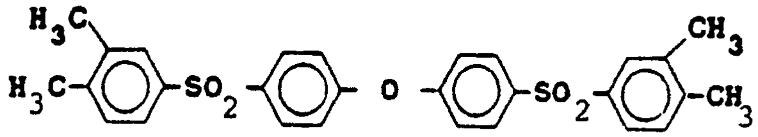

Then, 2 grams of the thus obtained DXDE were added to 20 milliliters of acetic acid. To this were added 0.2 gram of cobalt (II) acetate, 0.02 gram of manganese acetate and 0.1 gram of sodium bromide. The reaction was continued for 12 hours at a temperature of 100 °C with stirring while blowing oxygen into the reaction system.

Thereafter, the feeding of oxygen was stopped. The reaction mixture was cooled, and 100 milliliters of water were added to the resulting reaction mixture during which time a solid deposited. This solid was dissolved in a 10% aqueous solution of sodium hydroxide and to this aqueous solution was added hydrochloric acid to acidify it to obtain 1.8 gram of solid.

The resulting solid was recrystallized from a methanol/water mixture (6:4) to purify it. It was attempted to measure the melting point of the crystalline material but the dehydration/cyclization reaction occurred at a temperature of from 200 ° to 201 °C and thus the measurement of melting point was impossible.

The IR spectrum of the purified crystals was as follows:

1245 $cm^{-1}$: -O-; 1150 and 1320 $cm^{-1}$:

$-SO_2-$; 1485 and 1580 $cm^{-1}$: benzene rings; and 2200-3600 $cm^{-1}$: carboxyl groups.

Further, the results of elementary analysis were as follows:

Anal. calcd. for $C_{28}H_{18}O_{13}S_2$: C,

53.68%; H, 2.90%.

Found: C, 53.83%; H, 2.99%.

From these results, the resulting product was identified to be 4,4-bis(3,4-dicarboxyphenylsulfonyl)-diphenyl ether(DPDE) of the following structural formula:

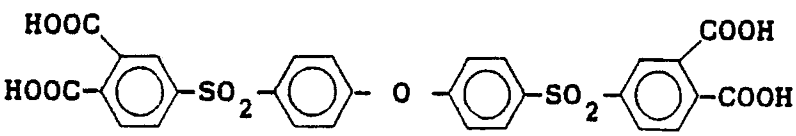

EXAMPLE 3

Two grams of DXDE obtained in Example 2, 20 milliliters of acetic acid, 0.2 gram of cobalt (II) acetate, 0.02 gram of manganese (II) acetate and 0.1 gram of sodium bromide were charged into a 100 ml autoclave, and the reaction was carried out for 6 hours under an air pressure of 10 $kg/cm^2$ at a temperature of 120 °C. During the reaction, an operation was repeated wherein the pressure was returned to atmospheric pressure and the air pressure of 10 $kg/cm^2$ was newly applied, several times. After the completion of the reaction, the same subsequent treatment as in Example 2 was carried out. As a result, DPDE crystals were obtained.

EXAMPLE 4

A titanium autoclave equipped with a reflux condenser, gas inlet and stirrer was charged with 15 grams of DXDE obtained in the same manner as in Example 2, 100 grams of acetic acid, 0.3 gram of cobalt acetate, 0.015 gram of manganese acetate and 0.25 gram of sodium bromide.

Nitrogen was introduced via the gas inlet to apply a pressure of 20 $kg/cm^2$, and thereafter the temperature was raised to 190 °C by means of a heater. After the temperature was raised to 190 °C, air was fed via the gas inlet to substitute nitrogen with air. Thereafter, the reaction was carried out for one hour while blowing air by portions.

The reaction product was cooled, and subjected to solid-liquid separation. It was washed with water, and 14.6 grams of the resulting solid were recrystallized from methanol/water mixture (6:4) to purify it. The resulting crystalline solid was identical with that identified in Example 2.

EXAMPLE 5

Two grams of 4,4'-bis(4-methyl-phenylsulfonyl)diphenyl ether obtained as described in Example 1 were than added to 20 milliliters of acetic acid. To this were added 0.2 gram of cobalt (II) acetate, 0.02 gram of manganese acetate, and 0.1 gram of sodium bromide. The reaction was continued for 12 hours at a temperature of 100 °C with stirring while blowing oxygen into the reaction system.

Thereafter, the feeding of oxygen was stopped and the reaction mixture was cooled. 100 milliliters of water were than added to the reaction mixture to deposit a solid. This solid was dissolved in a 10% aqueous solution of sodium hydroxide and hydrochloric acid was added to this aqueous solution to acidify it to obtain 1.8 gram of a solid.

The solid obtained was recrystallized from a methanol/water mixture (6:4) to purify it.

The melting point of the resulting crystals was measured by DTA. The DTA curve of these crystals was identical with that shown in Example 1.

Further, the IR analysis and elementary analysis of the resulting crystals were carried out. The results were identical with those of Example 1.

From these results, the crystals obtained in the reaction described above were confirmed to be 4,4'-bis-(4-carboxyphenylsulfonyl)diphenyl ether represented by the following formula:

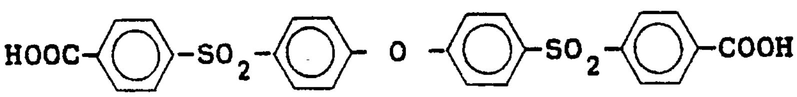

## Claims

1. A process for producing an aromatic carboxylic acid represented by the following formula [V]:

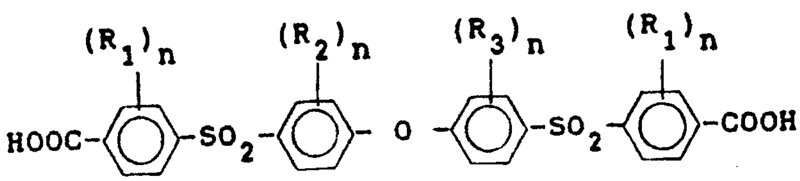

[V]

wherein $R_1$, $R_2$ and $R_3$, which may be the same or different, are hydrogen, a lower alkyl group containing from 1 to 6 carbon atoms or a carboxyl group; and n is the number of substituents other than hydrogen and is an integer of from 0 to 4, which process comprises the step of oxidizing a compound represented by the following general formula [IV]:

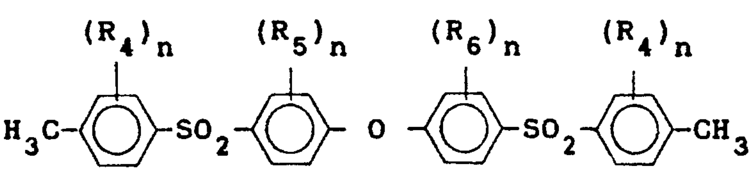

[IV]

wherein n is as defined above; $R_4$ is hydrogen or a methyl group; and $R_5$ and $R_6$, which may be the same or different, are hydrogen, or a lower alkyl containing from 1 to 6 carbon atoms, wherein the step of oxidizing is performed with molecular oxygen in a liquid phase in an aliphatic carboxylic acid solvent in the presence of a cobalt-manganese-bromine catalyst.

2. A process as claimed in claim 1 wherein said compound represented by formula [IV] is obtained by reacting a para-toluenesulfonyl chloride represented by the following general formula [II] with a diphenyl ether represented by the following formula [III]:

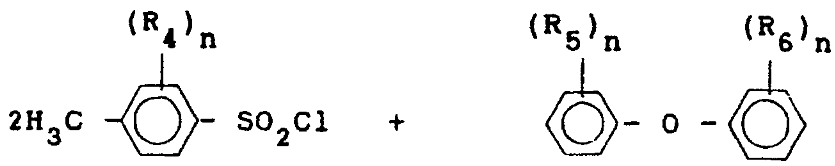

[II] [III]

wherein $R_4$, $R_5$,$R_6$ and n are as defined in claim 1.

3. A process as claimed in claim 2 wherein para-toluenesulfonyl chloride, in which in the general formula [II] $R_4$ is hydrogen, is reacted with diphenyl ether, in which in the general formula [III] $R_5$ and $R_6$ are each hydrogen.

4. A process as claimed in claim 2 or claim 3 wherein para-toluenesulfonyl chloride represented by the general formula [II] is reacted with a diphenyl ether having the general formula [III] in the presence of a Lewis acid.

5. Polyesters, polyamides and polyimides whenever prepared from a carboxylic acid produced by a process as claimed in any one of claims 1 to 4.

6. Manufactured articles such as synthetic fibres whenever made from a polyester, polyamide or polyimide as claimed in claim 5.

**Revendications**

1. Procédé de préparation d'un acide carboxylique aromatique répondant à la formule [V] suivante :

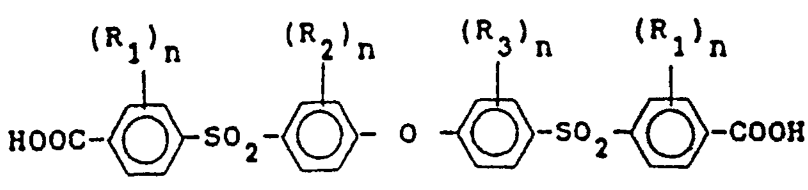

[V]

dans laquelle $R_1$, $R_2$ et $R_3$, qui peuvent être identiques ou différents, représentent un atome d'hydrogène, un groupe alcoyle inférieur comportant 1 à 6 atomes de carbone, ou un groupe carboxyle ; n est le nombre de substituants autres que l'hydrogène et représente un nombre entier valant 0 à 4, ledit procédé comprenant l'étape qui consiste à oxyder un composé répondant à la formule générale [IV] suivante :

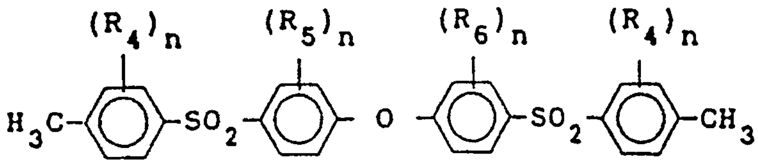

[IV]

dans laquelle n a la même définition que ci-dessus ; $R_4$ représente un atome d'hydrogène ou un groupe méthyle ; et $R_5$ et $R_6$, qui peuvent être identiques ou différents, représentent un atome d'hydrogène ou un alcoyle inférieur comportant 1 à 6 atomes de carbone,
caractérisé en ce que l'oxydation est réalisée avec de l'oxygène moléculaire en phase liquide dans un solvant d'acide carboxylique aliphatique, en présence d'un catalyseur de cobalt-manganèse-brome.

2. Procédé selon la revendication 1, caractérisé en ce qu'on obtient ledit composé répondant à la formule [IV] en faisant réagir un chlorure de para-toluènesulfonyle, répondant à la formule générale [II] suivante, avec un éther diphénylique répondant à la formule [III] suivante :

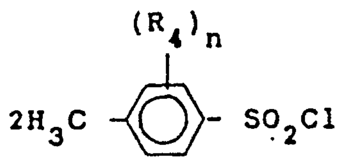

[II]

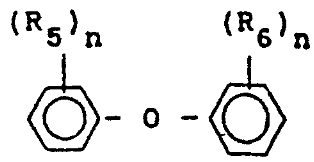

[III]

dans lesquelles $R_4$, $R_5$, $R_6$ et n ont la même définition que dans la revendication 1.

3. Procédé selon la revendication 2, caractérisé en ce qu'on fait réagir le chlorure de para-toluènesulfonyle, dans lequel $R_4$ représente un atome d'hydrogène dans la formule [II], avec un éther diphénylique, dans lequel $R_5$ et $R_6$ représentent chacun un atome d'hydrogène dans la formule [III].

4. Procédé selon la revendication 2 ou 3, caractérisé en ce qu'on fait réagir le chlorure de para-toluènesulfonyle, répondant à la formule générale [II], avec un éther diphénylique de formule générale [III], en présence d'un acide de Lewis.

5. Polyesters, polyamides et polyimides, caractérisés en ce qu'on les prépare à partir d'un acide carboxylique préparé au moyen d'un procédé selon l'une quelconque des revendications 1 à 4.

6. Articles manufacturés, tels que des fibres synthétiques, caractérisés en ce qu'on les réalise à partir d'un polyester, d'un polyamide ou d'un polyimide selon la revendication 5.

**Patentansprüche**

1. Verfahren zur Herstellung einer aromatischen Carbonsäure entsprechend der folgenden Formel [V]:

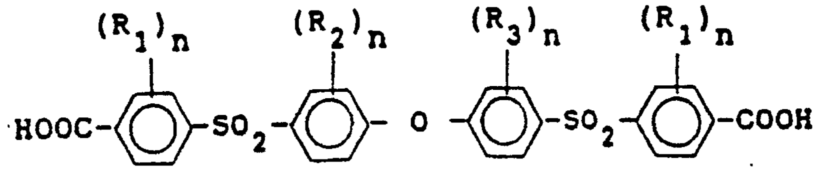

[V]

in der $R_1$, $R_2$ und $R_3$, die gleich oder verschieden sein können, Wasserstoff, eine Niedrigalkylgruppe mit 1 bis 6 Kohlenstoffatomen oder eine Carboxylgruppe bedeuten und n die Anzahl der Substituenten bedeutet, die von Wasserstoff verschieden sind und eine ganze Zahl von 0 bis 4 ist, bei dem man eine Verbindung entsprechend der folgenden allgemeinen Formel [IV] oxidiert:

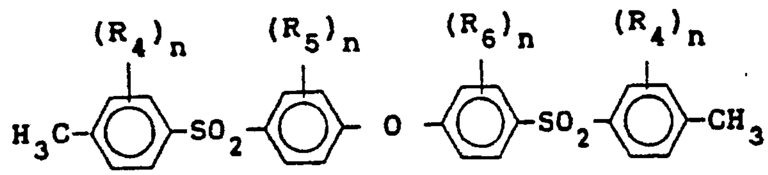

[IV]

in der n die oben gegebene Bedeutung besitzt; $R_4$ Wasserstoff oder eine Methylgruppe ist und $R_5$ und $R_6$, die gleich oder verschieden sein können, Wasserstoff oder eine Niedrigalkylgruppe mit 1 bis 6 Kohlenstoffatomen bedeuten, bei dem die Oxidation mit molekularem Sauerstoff in einer flüssigen Phase in einem aliphatischen Carbonsäure-Lösungsmittel in Gegenwart eines Cobalt-Mangan-Brom-Katalysators durchgeführt wird.

2. Verfahren nach Anspruch 1, bei dem die der Formel [IV] entsprechende Verbindung durch Reaktion eines para-Toluolsulfonylchlorids entsprechend der folgenden allgemeinen Formel [II] mit einem Diphenylether entsprechend der folgenden Formel [III] erhalten wird:

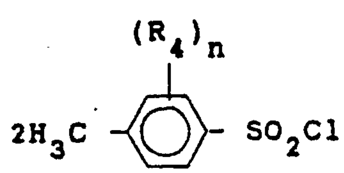

+

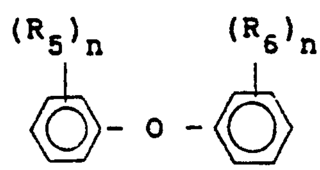

[II] [III]

in der $R_4$, $R_5$ und $R_6$ und n die in Anspruch 1 angegebene Bedeutung aufweisen.

3. Verfahren nach Anspruch 2, bei dem para-Toluolsulfonylchlorid in dem in der allgemeinen Formel [II] $R_4$ Wasserstoff bedeutet, mit Diphenylether, in dem in der allgemeinen Formel [III] sowohl $R_5$ als auch $R_6$ Wasserstoff sind, umgesetzt wird.

4. Verfahren nach Anspruch 2 oder Anspruch 3, bei dem para-Toluolsulfonylchlorid entsprechend der allgemeinen Formel [II] mit einem Diphenylether mit der allgemeinen Formel [III] in Gegenwart einer Lewissäure umgesetzt wird.

5. Polyester, Polyamide und Polyimide hergestellt aus einer Carbonsäure nach einem Verfahren gemäß einem der Ansprüche 1 bis 4.

6. Erzeugnis wie synthetische Fasern, hergestellt aus einem Polyester, Polyamid oder Polyimid wie in Anspruch 5 beansprucht.

FIG. I

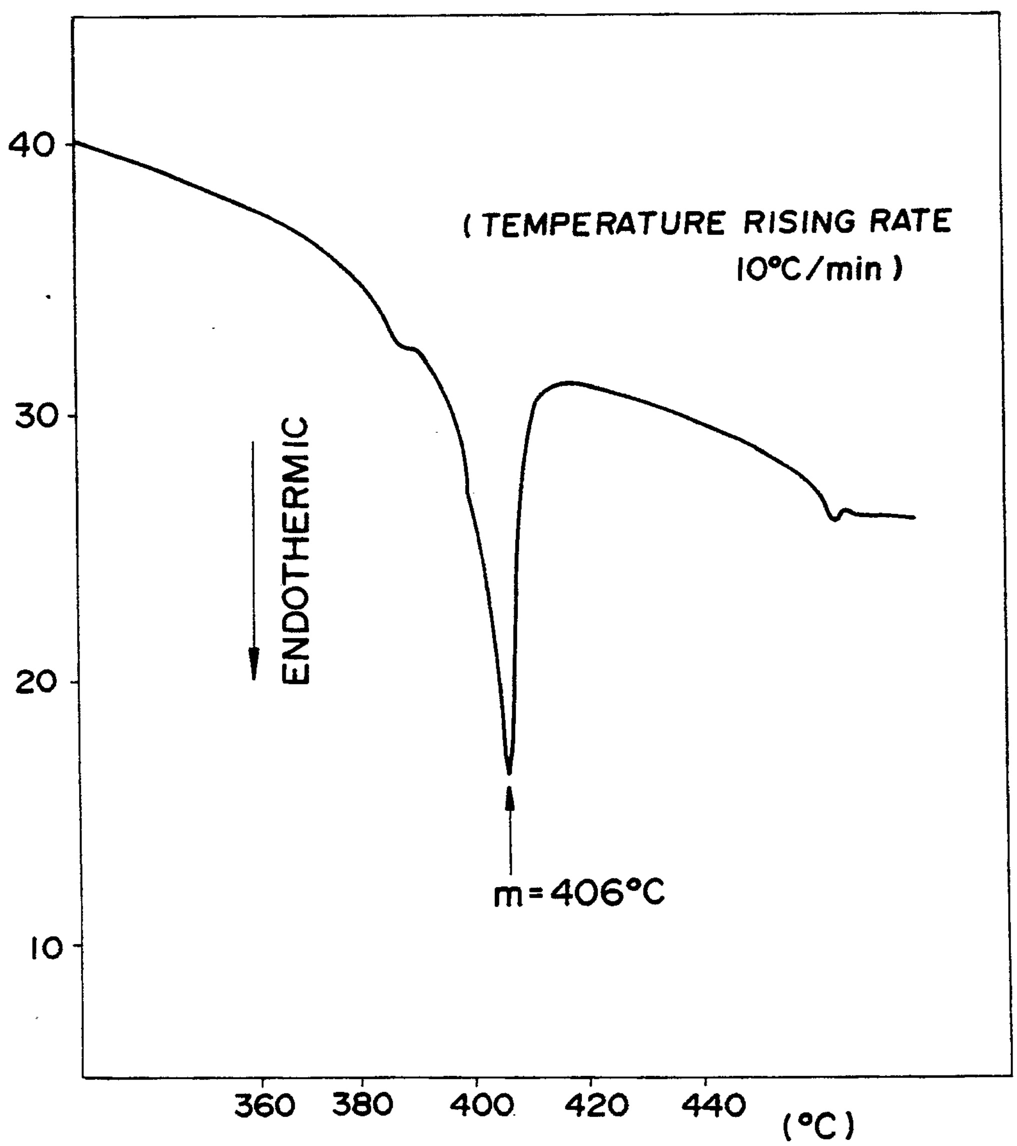
40
30
20
10
(TEMPERATURE RISING RATE
10°C/min)
ENDOTHERMIC
m=406°C
360
380
400
420
440
(°C)

FIG. 2